(19) 

(11) **EP 4 506 049 B1**

(12) **EUROPEAN PATENT SPECIFICATION**

(45) Date of publication and mention of the grant of the patent: **02.09.2026 Bulletin 2026/36**

(21) Application number: **24161745.5**

(22) Date of filing: **06.03.2024**

(51) International Patent Classification (IPC): ***B01D 19/04*** *(2006.01)* ***C08G 65/48*** *(2006.01)*

(52) Cooperative Patent Classification (CPC): **B01D 19/0404; C08G 65/3322**

(54) **AN ANTIFOAM / DEFOAM FORMULATION**

ANTISCHAUM-/ENTSCHAUMFORMULIERUNG

FORMULATION ANTIMOUSSE/DÉMOUSSANTE

(84) Designated Contracting States: **AL AT BE BG CH CY CZ DE DK EE ES FI FR GB GR HR HU IE IS IT LI LT LU LV MC ME MK MT NL NO PL PT RO RS SE SI SK SM TR**

(30) Priority: **08.08.2023 IN 202341053242**

(43) Date of publication of application: **12.02.2025 Bulletin 2025/07**

(73) Proprietor: **Hindustan Petroleum Corporation Limited**
**Bangalore 560067 (IN)**

(72) Inventors:
- **MUKHERJEE, Sanjoy**
  **560067 Bengaluru (IN)**
- **BHOWMIK, Sandip**
  **560067 Bengaluru (IN)**
- **CHINTHALAPATI, Siva Kesava Raju**
  **560067 Bengaluru (IN)**
- **JAINENDRAKUMAR, Shankar**
  **560067 Bengaluru (IN)**
- **SRINIVASA NARASIMHA, Sheshachala**
  **560067 Bengaluru (IN)**
- **BALASUBRAMANIAM, Ravi**
  **530011 Visakhapatnam (IN)**

(74) Representative: **Petty, Catrin Helen et al**
**Venner Shipley LLP**
**St James's**
**79 Oxford Street**
**Manchester M1 6EG (GB)**

(56) References cited:
**US-A- 2 965 581**

- **WU YAOBIN ET AL: "Injectable biodegradable hydrogels and microgels based on methacrylated poly(ethylene glycol)-co-poly(glycerol sebacate) multi-block copolymers: synthesis, characterization, and cell encapsulation", JOURNAL OF MATERIALS CHEMISTRY. B, vol. 2, no. 23, 1 January 2014 (2014-01-01), GB, pages 3674, XP093193980, ISSN: 2050-750X, DOI: 10.1039/c3tb21716g**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 506 049 B1

## Description

### FIELD OF THE INVENTION

The present invention relates to an antifoaming / defoaming compound having effects of breaking foam and its process of preparation thereof. The present invention also relates to an antifoaming formulation having an additive and the promoter.

### BACKGROUND OF THE INVENTION

According to the classical conceptions, the foams are dispersed systems in which the disperse part is gaseous, but the dispersant is liquid. Numerous industrial processes involving a treatment step of liquid or a treatment step with liquid is often associated with problems of foam formation. These foaming issues are often heightened in the presence of soluble metal salts and insoluble salt particulates, other organic constituents and containments. The foaming of the liquid is contributory of a reduction in the efficiency of the treatment process or the quality of a product.

To mitigate foaming related efficiency losses, additives are used in industrial processes.

Additives such as antifoaming / defoaming agents having effects of breaking foam. i.e., a foam breaking effect, or an effect of lowering the foam formation, i.e., a foam controlling effect is used to solve the difficult foaming of liquid. The mechanistic essence of additive based foam control is that the antifoam agent itself accumulates at the air-liquid interface, disrupts the foams stability, but is unable to form a new, ordered, monomolecular film layer. Commonly used anti-foaming agents are water insoluble oils, such as silicones, certain alcohols, stearates and glycols.

Antifoaming / defoaming additives are often emulsified mixtures of poly-ethers and fatty acids, and / or fatty acid esters. Many additive formulations are fatty acid based emulsion products. Some examples of fatty acid based emulsion products are set forth in the following prior arts.

US3705860A relates to an improved antifoam agents particularly adapted for use in paper and pulp manufacturing processes and prepared from a mixture of non-polar oil, precipitated silica, metallo-organic catalyst, polymethylsiloxane and microcrystalline paraffin wax. These antifoam agents are useful also in latex paint manufacture.

US3337595A relates to new compounds, i.e., fatty acid esters of polyoxypropylated glycerol. These compounds have proven useful for controlling, suppressing and/or preventing foaming of aqueous systems having foaming tendencies.

DE3013292 relates to a process for preparing anti-foams in a free-flowing form which, because of their low melting or softening point, tend to cake when stored.

US4451390A relates to a composition for the control of unwanted foaming comprising Soya bean oil, a mineral oil, a finely divided silica, an unsubstituted fatty acid monoester of glycerol, an unsubstituted fatty acid ester of a polyoxyalkylated sorbitan. These compositions are valuable for the control of foam especially in fermentations since they are stable to sterilization. Also, they may be used in paints since they do not give the defects often associated with silicone antifoams.

US2965581A discloses an anti-foam composition based on fatty acid, a di-alcohol and fatty di-acid.

Antifoaming / defoaming additives are often emulsified mixtures of poly-ethers and fatty acids, and / or fatty acid esters. Many additive formulations are fatty acid-based emulsion products. Linear organic esters containing two chemically dissimilar hydrophilic and hydrophobic chain-ends are favourable as they can be tailored to be chemically robust yet non-corrosive in nature.

### OBJECTIVE OF THE INVENTION

The primary objective of the present invention is to provide an antifoaming formulation that doesn't suffer from phase-separation, easing handling and usage restrictions.

An anti-foaming formulation is defined in claim 1 and a process for its use is defined in claim 3.

### SUMMARY OF THE INVENTION

In the present invention a series of fatty-acid esters of poly-oxyethylene is provided by gradually altering the fatty-acid chain length in a series of fatty acids polyol esters. The selected fatty-acids were further taken for preparing alternative tri-block (A-B-A) and penta-block (A-B-C-B-A) chains to find their relative efficacy. Finally, a tetra-arm $[A'-B']_4C'$ type molecule are prepared, not part of the invention, which can further enhance disruption of foaming tendencies due to its non-linear architecture. All the designs could be achieved using scalable esterification reactions. Due to chemical tethering of the fatty-acid onto the polyol chains, the resultant liquid materials do not suffer from phase-separation, easing handling and usage restrictions.

In a first aspect of the present disclosure, there is provided an antifoaming promoter having structural formula (I) or (Ia):

Linear A-B-C-B-A (I);

or

Tetra-arm **[A'-B']$_4$C'** **(Ia)**

wherein:

A is moiety from fatty acid,
B is moiety from di-alcohol
C is moiety from fatty di-acid.
A' is moiety from fatty alcohol,
B' is moiety from fatty di-acid, and
C' is moiety from tetra alcohol.

The present invention provides antifoaming promoter, wherein the fatty acid is selected from myristic acid, stearic acid and hexadecenoic acid; the di-alcohol is selected from PEG-400 and polypropylene glycol; the fatty di-acid is selected from sebacic acid, adipic acid and suberic acid.

The antifoaming promoter formula (I), wherein the formula (I) is optionally compound 9:

Compound 9

A method of synthesizing the compound 9 by a process comprising steps of:

(a) adding of compound 8 and myristic acid in toluene to obtain a reaction mixture;

compound 8

(b) heating the reaction mixture of step (a) with constant stirring for 4 to 8 hours to obtain compound 9.

The heating of reaction mixture in synthesis of compound 9 is carried out at a temperature of 110°C.

A method of synthesizing the compound 8 by a process comprising steps of:

(a) adding sebacic acid and PEG-400 in toluene to obtain a reaction mixture; and
(b) heating the reaction mixture with constant stirring for 4 to 8 hours to obtain compound 8.

The heating of reaction mixture in synthesis of compound 8 is carried out at a temperature of 110°C.

Another antifoaming promoter of Tetra-arm [A'-B']$_4$C' structure, not part of the invention, is disclosed wherein the fatty alcohol is selected from myristyl alcohol, stearyl alcohol and dodecanol; the fatty di-acid is selected from sebacic acid, adipic acid and suberic acid; the tetra alcohol is selected from pentaerythritol, erythritol and threitol.

The antifoaming promoter formula (Ia), wherein the formula (Ia) is compound 10:

Compound 10

A method of synthesizing antifoaming compound 10 by a process comprising steps of:

(a) adding sebacic acid and myristyl alcohol in toluene to obtain a reaction mixture;
(b) heating the reaction mixture of step (a) with constant stirring for 4 to 8 hours; and
(c) adding pentaerythritol in the reaction mixture of step (b) and continuing stirring for 8 to 16 to obtain compound 10.

The heating of reaction mixture in synthesis of compound 10 is carried out at a temperature of 110°C.
The present invention provides an antifoaming formulation comprising:

(a) an additive, wherein the additive is compound 4

;

and
(b) the promoter as disclosed herein.

Additionally, the present invention provides a process for foam reduction which comprises incorporating the antifoaming formulation in a material generating the foam, wherein the amount of formulation is from 1 to 20 ppm of the material generating the foam.

## BRIEF DESCRIPTION OF THE FIGURES

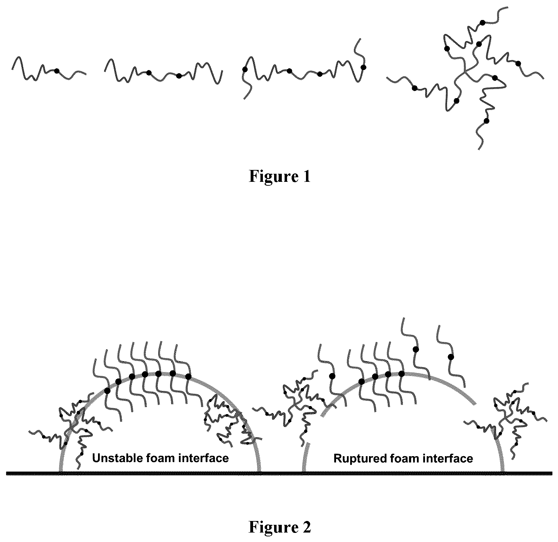

**Figure 1:** illustrates schematic representation of the architectures of synthesized compounds. From left to right: linear di-block A-B type, linear tri-block A-B-A type, linear penta-block A-B-C-B-A type, and tetra-arm $[A'-B']_4C'$ type designs.

**Figure 2:** illustrates plausible mechanism of foam rupture in presence of additive and promoter.

**Figure 3:** illustrates experimental design for calculation of foam reduction efficiency of additive and additive in the presence of promoter in comparison with blank solution.

## DETAILED DESCRIPTION OF THE INVENTION

For the purpose of promoting an understanding of the principles of the invention, reference will now be made to the embodiments in the specific language will be used to describe the same. Unless otherwise defined, all technical and scientific terms used herein have the same meaning as commonly understood by one of ordinary skilled in the art to which this invention belongs. The composition, methods, and examples provided herein are illustrative only and not intended to be limiting.
The articles "a", "an" and "the" are used to refer to one or to more than one (i.e., to at least one) of the grammatical object of the article.
The terms "comprise" and "comprising" are used in the inclusive, open sense, meaning that additional elements may be included. It is not intended to be construed as "consists of only".

Throughout this specification, unless the context requires otherwise the word "comprise", and variations such as "comprises" and "comprising", will be understood to imply the inclusion of a stated element or step or group of element or steps but not the exclusion of any other element or step or group of element or steps.

Unless defined otherwise, all technical and scientific terms used herein have the same meaning as commonly understood by one of ordinary skill in the art to which this disclosure belongs. Although any methods and materials similar or equivalent to those described herein can be used in the practice or testing of the disclosure, the preferred methods and materials are now described. All publications mentioned herein are incorporated herein by reference.

In the present invention linear di-block esters of a poly-ether derivative and a fatty-acid derivative were synthesised using 1:1 molar ratio of the constituents, refluxed in toluene as a solvent at 110 °C.

Linear tri-block esters where the central block is a poly-ether derivative were synthesized using 1:2 molar ratios of the polyether and fatty-acid as constituents, refluxed in toluene as a solvent at 110°C.

Linear tri-block esters where the central block ester is a linear di-acid was prepared using 1:2 molar ratios of the di-acid and polyether, refluxed in toluene at 110 °C.

The same could be used for further esterification of the poly-ether open chain ends, to prepare linear penta-block esters. Di-block linear esters of di-acids and fatty-alcohols, when coupled with pentaerythritol under similar reaction conditions, yielded the tetra-arm derivatives (e.g., compound 10).

**Chart I**. Structures of compounds **1-10** of the present disclosure.

**Table 1: Structural Formula of synthesized compounds 1- 10**

| Structure-Formula | Polyol | Alkyl-acid component | Name |
|---|---|---|---|

| Linear A-B | PEG-400 (A) | Butanoic acid (B) | 1 |
|---|---|---|---|
| | PEG-400 (A) | Heptanoic acid (B) | 2 |
| | PEG-400 (A) | 2-ethyl hexanoic acid (B) | 3 |
| | PEG-400 (A) | Myristic acid (B) | 4 |
| | PEG-400 (A) | Stearic acid (B) | 5 |
| Linear A-B-A | PEG-400 (B) | Myristic acid (A) | 6 |
| | PEG-400 (B) | Sebacic acid (A) | 7 |
| | PEG-400 (A) | Sebacic acid (B) | 8 |
| Linear A-B-C-B-A | PEG-400 (B) | Sebacic (C), and Myristic acid (A) | 9 |
| Tetra-arm $[A'-B']_4C'$ | Pentaerythritol (C') | Sebacic (B'), and Myristyl alcohol (A') | 10 |

## EXAMPLES

The present disclosure with reference to the accompanying examples describes the present invention. It is understood that the examples are provided for the purpose of illustrating the invention only and are not intended to limit the scope of the invention in any way other than claimed.

**Example 1: Synthesis of compounds 1-5.**

40 g of PEG-400 (100 mmol) and 8.81 g of butyric acid (100 mmol) were taken with 100 mL toluene in a 250 mL round-bottom flask. The reaction mixture was heated to 110°C under constant stirring for 8 hours and the released water was separated from the reflux media using a dean-stark trap. After completion of the reaction, solvent was evaporated under vacuum, and the desired product (1) was obtained a colourless liquid (yield > 99%). Similar procedure was followed for compounds 2-5 where the 100 mmol of the corresponding fatty-acid was taken instead of butyric-acid. All compounds were obtained as colourless liquids **(Table 1).**

110 deg C / Toluene

**Scheme 1**

where R is any linear or branched alkyl group,
and n ranges from 10-25.

**Example 2: Synthesis of compounds 6 and 7.**

40 g of PEG-400 (100 mmol) and 45.70 g of myristic acid (200 mmol) were taken with 100 mL toluene in a 250 mL round-bottom flask. The reaction mixture was heated to 110°C under constant stirring for 8 hours and the released water was separated from the reflux media using a dean-stark trap. After completion of the reaction, solvent was evaporated under vacuum, and the desired product (6) was obtained a colourless liquid (yield > 99%). Similar procedure was followed for compound 7 where the 200 mmol of the corresponding di-acid was taken instead of myristic acid. Compound 7 was isolated as white coloured solid **(Table 1).**

Scheme 2

where R is any linear alkyl group, and n ranges from 10-25.

**Example 3: Synthesis of compound 8.**

20.22 g of sebacic acid (100 mmol) and 80 g of PEG-400 (200 mmol) were taken with 100 mL toluene in a 250 mL round-bottom flask. The reaction mixture was heated to 110°C under constant stirring for 8 hours and the released water was separated from the reflux media using a dean-stark trap. After completion of the reaction, solvent was evaporated under vacuum, and the desired product (8) was obtained as colourless liquid (yield > 98%) **(Table 1).**

Scheme 3

where n ranges from 10-25, and m is 8.

**Example 4: Synthesis of compound 9.**

96.6 g of compound 8 (100 mmol) and 45.70 g of myristic acid (200 mmol) were taken with 100 mL toluene in a 250 mL round-bottom flask. The reaction mixture was heated to 110°C under constant stirring for 8 hours and the released water was separated from the reflux media using a dean-stark trap. After completion of the reaction, solvent was evaporated under vacuum, and the desired product (9) was obtained as colourless liquid (yield > 97%) **(Table 1).**

**$^1$H NMR (500 MHz, $CDCl_3$):** σ 4.2 (m, 8H), 3.7 (m, 40H), 2.4 (m, 8H), 1.7 (m, 8H), 1.3 (m, 48H), 0.9 (t, 6H). FTIR 2920 $cm^{-1}$, 2850 $cm^{-1}$, 1735 $cm^{-1}$, 1450 $cm^{-1}$, 1350 $cm^{-1}$, 1250 $cm^{-1}$, 1100 $cm^{-1}$, 725 $cm^{-1}$, 695 $cm^{-1}$.

Scheme 4

where n ranges from 10-25, and m is 8.

**Example 5: Synthesis of compound 10.**

20.22 g of sebacic acid (100 mmol) and 21.4 g of myristyl alcohol (100 mmol) were taken with 100 mL toluene in a 250 mL round-bottom flask. The reaction mixture was heated to 110°C under constant stirring for 8 hours and the released water was separated from the reflux media using a dean-stark trap. Further, 3.4 g of pentaerythritol (25 mmol) was added in the reaction mixture and the reaction was continued for another 16 hrs. After completion of the reaction, the solvent was evaporated, and the desired compound was obtained as a low melting white-coloured solid **(Table 1).**

**$^1$H NMR (500 MHz, $CDCl_3$):** σ 4.25 (t, 8H), 3.7 (m, 8H), 2.3 (m, 16H), 1.6 (m, 24H), 1.3 (m, 120H), 0.9 (t, 12H). FTIR 2950 $cm^{-1}$, 2915 $cm^{-1}$, 2850 $cm^{-1}$, 1731 $cm^{-1}$, 1700 $cm^{-1}$, 1420 $cm^{-1}$, 1300 $cm^{-1}$, 1215 $cm^{-1}$, 1175 $cm^{-1}$, 715 $cm^{-1}$.

Scheme 5

**Example 6: Evaluation of the formulations.**

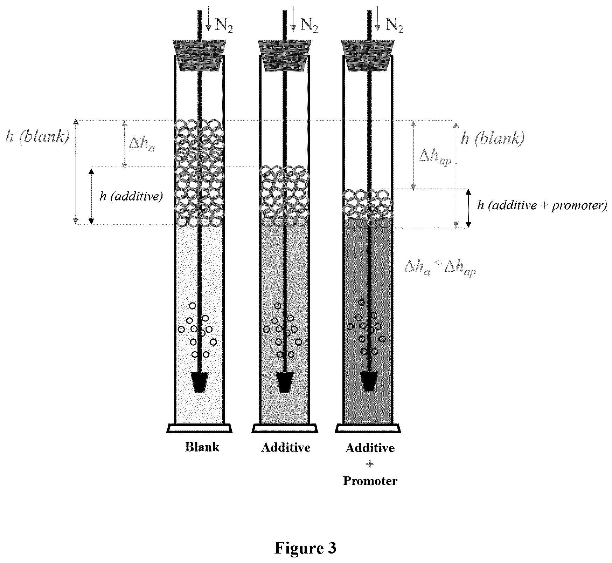

The foaming tests were carried out by measuring equilibrium foam heights. For example, a blank / reference and an additive containing test solutions were exposed to a constant purge of $N_2$ (nitrogen) and the equilibrium heights were compared to determine the percentage of foam reduction as an effect of the additive. A solution of 35 weight % MDEA (methyl di-ethanol amine) with 0.5 weight % $Fe(NO_3)_3 \cdot 9H_2O$ (iron nitrate nonahydrate) was taken as the reference / blank solution (Figure 3 & Table 2).

Equal volume of a blank solution / reference solution is compared with an additive containing solution under equilibrium nitrogen ($N_2$) purging i.e., all the conditions in two parallel set-ups are maintained same except that one of the solutions contain the additive formulation.

The foam-reduction is calculated as follows,

$$\%\ \text{Foam reduction} = \Delta h \div h\ (blank) \times 100$$

**Table 2: The % Foam reduction obtained for the additives and promoter**

| Additive composition | | Dosage | % Foam Reduction |
|---|---|---|---|
| **Additive** | **Promoter** | | |
| Compound 1 (100%) | - | 5 ppm | Negligible |
| Compound 2 (100%) | - | 5 ppm | Negligible |
| Compound 3 (100%) | - | 5 ppm | ~60% |
| **Compound 4 (100%)** | - | 5 ppm | ~90% |
| **Compound 4 (100%)** | - | 1 ppm | ~85% |
| Compound 5 (100%) | - | 5 ppm | ~70% |
| Compound 4 (95%) | Compound 6 (5%) | 5 ppm | ~90% |

| Compound 4 (95%) | Compound 7 (5%) | 5 ppm | ~90% |
|---|---|---|---|
| Compound 4 (95%) | Compound 8 (5%) | 5 ppm | ~90% |
| Compound 4 (95%) | Compound 9 (5%) | 5 ppm | >90% |
| **Compound 4 (95%)** | **Compound 10 (5%)** | 5 ppm | >95% |
| **Compound 4 (95%)** | **Compound 10 (5%)** | 1 ppm | >90% |

Comprehensive screening of the different additives helped to identify the best additive (i.e., Compound 4) which is further taken up with different promoters to find the best possible formulation (i.e., Compound 4 and Compound 10 in 19:1 ratio). The tetra-arm designs act as performance enhancers for linear systems as the non-linear structure would destabilize the foam surfaces to greater effects. The finalized formulations could reduce the foaming of the media up to 95% extent when compared to reference solutions.

## Claims

**1.** An antifoaming formulation comprising:

(a) an additive, wherein the additive is compound 4:

compound 4;

and
(b) a promoter having structural formula (I):

Linear A-B-C-B-A (I);

wherein:

A is a moiety derived from a fatty acid selected from the group consisting of myristic acid, stearic acid, and hexadecenoic acid,
B is a moiety derived from a di-alcohol selected from the group consisting of PEG-400 and polypropylene glycol, and
C is a moiety derived from a fatty di-acid selected from the group consisting of sebacic acid, adipic acid, and suberic acid.

**2.** The antifoaming formulation as claimed in claim 1, wherein the promoter is compound 9:

Compound 9

**3.** A process for foam reduction comprising incorporating the antifoaming formulation as claimed in claim 1 in a material generating the foam, wherein the antifoaming formulation is present in an amount ranging from 1 to 20 ppm based on the material generating the foam.

**Patentansprüche**

**1.** Antischaumformulierung, umfassend:

(a) ein Additiv, wobei das Additiv Verbindung 4 ist:

Verbindung 4;

und
(b) einen Promotor mit der Strukturformel (I):

Linear A-B-C-B-A (I);

wobei:

A ist eine Einheit ist, die von einer Fettsäure abgeleitet ist, die aus der Gruppe ausgewählt ist, die aus Myristinsäure, Stearinsäure und Hexadecensäure besteht,
B eine Einheit ist, die von einem Di-Alkohol abgeleitet ist, der aus der Gruppe ausgewählt ist, die aus PEG-400 und Polypropylenglycol besteht, und
C eine Einheit ist, die von einer Fett-Disäure abgeleitet ist, die aus der Gruppe ausgewählt ist, die aus Sebacinsäure, Adipinsäure und Korksäure besteht.

**2.** Antischaumformulierung nach Anspruch 1, wobei der Promotor Verbindung 9 ist:

Verbindung 9.

**3.** Verfahren zur Schaumreduktion, umfassend Einschließen der Antischaumformulierung nach Anspruch 1 in ein den Schaum erzeugendes Material, wobei die Antischaumformulierung in einer Menge im Bereich von 1 bis 20 ppm, bezogen auf das den Schaum erzeugende Material, vorliegt.

**Revendications**

**1.** Formulation antimousse comprenant :

(a) un additif, dans laquelle l’additif est le composé 4 :

composé 4 ;

et
(b) un promoteur présentant la formule structurale (I) :

A-B-C-B-A linéaire (I) ;

où :

A est un fragment dérivé d'un acide gras choisi dans le groupe constitué par l'acide myristique, l'acide stéarique et l'acide hexadécénoïque,
B est un fragment dérivé d'un dialcool choisi dans le groupe constitué par le PEG-400 et le polypropylène glycol, et
C est un fragment dérivé d'un diacide gras choisi dans le groupe constitué par l'acide sébacique, l'acide adipique et l'acide subérique.

2. Formulation antimousse selon la revendication 1, dans laquelle le promoteur est le composé 9 :

Composé 9.

3. Processus permettant la réduction de mousse comprenant l'incorporation de la formulation antimousse selon revendication 1 dans un matériau générant de la mousse, dans lequel la formulation antimousse est présente en une quantité comprise entre 1 et 20 ppm sur la base du matériau générant de la mousse.

Figure 1

Unstable foam interface
Ruptured foam interface

Figure 2

$N_2$
$N_2$
$N_2$
h (blank)
$\Delta h_a$
h (additive)
$\Delta h_{ap}$
h (blank)
h (additive + promoter)
$\Delta h_a < \Delta h_{ap}$
Blank
Additive
Additive + Promoter


**Figure 3**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 3705860 A
- US 3337595 A
- DE 3013292
- US 4451390 A
- US 2965581 A